(19) Europäisches Patentamt European Patent Office Office européen des brevets

(11) **EP 4 738 373 A1**

(12) **EUROPEAN PATENT APPLICATION**

(43) Date of publication:
**06.05.2026 Bulletin 2026/19**

(21) Application number: **25212595.0**

(22) Date of filing: **31.10.2025**

(51) International Patent Classification (IPC):
**G16H 10/40** (2018.01)    **G16H 50/20** (2018.01)
**G16H 50/70** (2018.01)

(52) Cooperative Patent Classification (CPC):
**G16H 50/20; G16H 10/40; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **01.11.2024 JP 2024192741**

(71) Applicant: **FUJITSU LIMITED**
**Kawasaki-shi, Kanagawa 211-8588 (JP)**

(72) Inventor: **MURAKAMI, Katsuhiko**
**Kawasaki-shi, 211-8588 (JP)**

(74) Representative: **Haseltine Lake Kempner LLP**
**Cheapside House**
**138 Cheapside**
**London EC2V 6BJ (GB)**

(54) **DRUG EFFICACY PREDICITNG PROGRAM, METHOD FOR PREDICTING DRUG EFFICACY, AND DRUG EFFICACY PREDICTING DEVICE**

(57)    A drug efficacy predicting program for causing a computer to execute a process including: predicting, by using a first machine learning model, a difference in drug efficacy between one or more of two data combinations under a same experimental system, each of the two data combinations including at least one common item among items of a drug and a cell line; and predicting, by using a second machine learning model, one or more drug efficacy of each of two or more data using the predicted difference.

## FIG.7

## Description

[Technical Field]

The embodiments discussed herein are related to a drug efficacy predicting program, a method for predicting drug efficacy, and a drug efficacy predicting device.

[Background Art]

For example, the field of genome drug discovery has demanded an efficiently search for which compounds (new drug candidates) are likely to be effective to which type of cancer.

As a numeric value representing a degree of drug efficacy of a drug to a certain cell line, IC50 value has been used. For example, in drug discovery for a particular cancer type, an IC50 has been used to select drug candidates (compounds). An IC50 is used as a numeric value representing drug efficacy.

Since it is very difficult to measure IC50s under all conditions, features were learned from measured data (a set of IC50 values and features) and the IC50 under an unknown condition was inferred.

In addition, a study has been known which applies deep learning (DL) to predict drug efficacy.

For example, data of different experimental systems using, for example, different solvents are not considered to be directly comparable because it is difficult to adjust their values. Accordingly, drug efficacy needs to be examined for each individual experimental system. Specifically, a particular experimental system is fixed as a condition, and a machine learning model is prepared for each individual experimental system. Using the machine learning model, drug efficacy is inferred.

As an example of features (explanatory variables), expression level (over 10,000 variables) of genes is used for a certain cell line. In addition, the chemical structure of a drug is used as a feature. The prediction of the drug efficacy is treated as a regression problem, using numeric IC50 values as target values in the training data.

[Prior Art Reference]

[Non-Patent Document]

    [Patent Document 1] Japanese Laid-open Patent Publication No. 2021-144619
    [Patent Document 2] Japanese Laid-open Patent Publication No. 2021-39565
    [Patent Document 3] US Patent Application Publication No. 2011/0173144
    [Patent Document 4] Japanese Laid-open Patent Publication No.2019-125045
    [Patent Document 5] US Patent Application Publication No. 2020/0175380

[Summary of Invention]

[Problems to be Solved by Invention]

However, since such a conventional method for predicting drug efficacy is unable to directly compare drug efficacy (IC50s) of the different experimental systems, data of the different experimental systems cannot be collectively learned in the same machine learning model. Accordingly, the conventional method makes use only part of the data for prediction of drug efficacy and therefore has difficulty in accurately predicting the drug efficacy. Normally, the same experimental system is used in the same project, but different experimental systems are used between different projects. Since it has not been normally assumed that data are compared across projects, the experimental systems are selected on a case-by-case basis and therefore different projects use respective different experimental systems. Ideally, if the above main conditions are the same, it is expected that IC50s representing drug efficacy are approximately the same under different experimental systems. However, the actual IC50 values considerably deviate from one another, so that it has been considered that the IC50 values of different experimental systems are not directly compared. Therefore, it has been difficult to collectively treat the IC50 values of different experimental systems.

Accordingly, it is desirable to accurately predict drug efficacy.

[Means to Solve the Problem]

According to an aspect of the embodiments, a drug efficacy predicting program causes a computer to execute a process including: predicting, using a first machine learning model, a difference in drug efficacy between one or more of two data combinations under a same experimental system, wherein input explanatory variables are jointly formed by feature vectors of the two data combinations, each of the two data combination including at least one common item among items of a drug and a cell line; and predicting, by using a second machine learning model, one or more drug efficacies of two or more data using the predicted difference.

[Effect of Invention]

Prediction of drug efficacy can be performed accurately.

[Brief Description of Drawings]

The invention is described, by way of example only, with reference to the following drawings, in which:

FIG. 1 is a diagram illustrating an example of a functional configuration of a drug efficacy predicting device according to one embodiment;
FIG. 2 is a block diagram illustrating an example of a hardware (HW) configuration of a computer that achieves the functions of the drug efficacy predicting device according to the embodiment;
FIG. 3 is a diagram illustrating a first data set of the drug efficacy predicting device according to the embodiment;
FIG. 4 is a diagram illustrating a second data set of the drug efficacy predicting device according to the embodiment;
FIG. 5 is a diagram illustrating a third data set of the drug efficacy predicting device according to the embodiment;

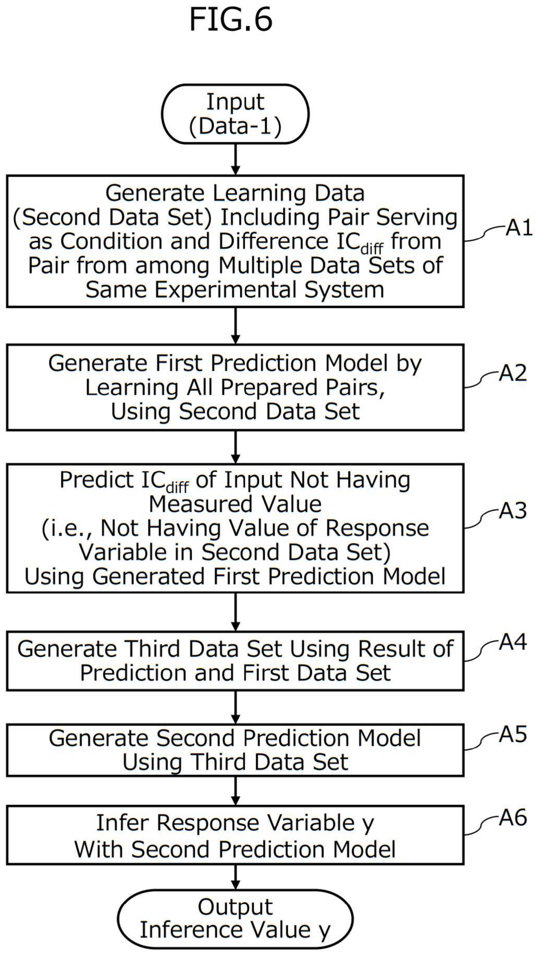

FIG. 6 is a flow chart illustrating a process performed in the drug efficacy predicting device according to the one embodiment;
FIG. 7 is a diagram illustrating an overview of a process performed in the drug efficacy predicting device according to the one embodiment;
FIG. 8 is a diagram illustrating an example of a state where the first data set is divided into multiple regions; and
FIG. 9 is a flow chart illustrating a process performed in the drug efficacy predicting device according to a modification to the one embodiment.

[Description of Embodiments]

Hereinafter, the drug efficacy predicting program, the method for predicting drug efficacy, and the drug efficacy predicting device according to one embodiment will now be described with reference to the accompanying drawings. However, the following embodiment is merely illustrative and is not intended to exclude the application of various modifications and techniques not explicitly described in the embodiment. For example, the present embodiment can be variously modified and implemented without departing from the scope thereof (by, for example, combining the embodiment and respective modifications). Further, each of the drawings can include additional functions not illustrated therein to the elements illustrated in the drawing.
"Drug efficacy may be represented, for example, by IC50 values, EC50 values, AUC values, or other quantitative measures."

Configuration:

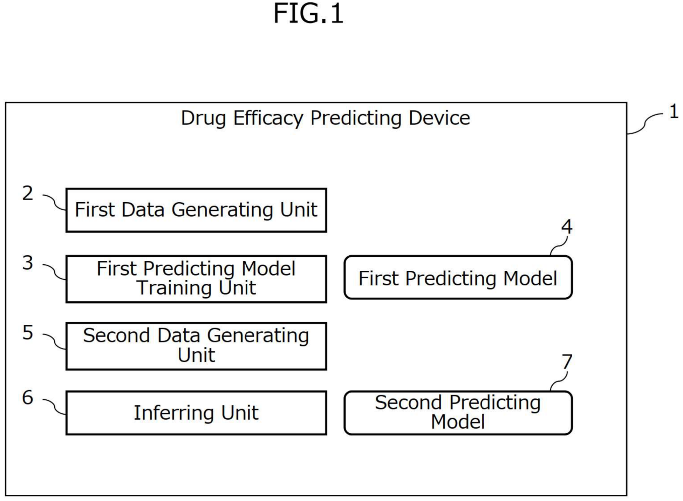

FIG. 1 is a diagram schematically illustrating a functional configuration of a drug efficacy predicting device 1 according to one embodiment.
The drug efficacy predicting device 1 predicts drug efficacy of a compound (drug). In the present embodiment, the drug efficacy predicting device 1 predicts an IC50 as an index representing drug efficacy.

Example of Hardware Configuration:

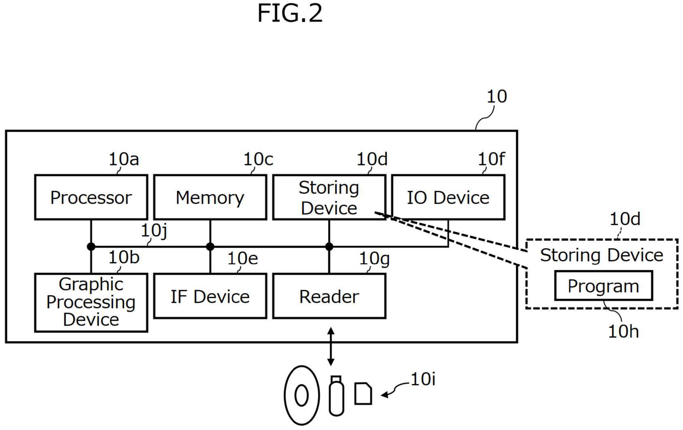

FIG. 2 is a block diagram illustrating an example of a hardware (HW) configuration of the computer 10 that achieves the functions of the drug efficacy predicting device 1 according to the one embodiment. If multiple computers are used as the HW resources for achieving the functions of the drug efficacy predicting device 1, each of the computers may include the HW configuration illustrated in FIG. 2.
As illustrated in FIG. 2, the computer 10 may illustratively include, as the HW configuration, a processor 10a, a graphic processing device 10b, a memory 10c, a storing device 10d, an Interface (IF) device 10e, an Input/Output (IO) device 10f, and a reader 10g.
The processor 10a is an example of an arithmetic processing device that performs various types of control and calculations and serves as a controller that carries out various processes. The processor 10a may be mutually communicably connected to each of the blocks in the computer 10 via a system bus 10j. The processor 10a may be a multi-processor including multiple processors or a multi-core processor including multiple processor cores, or may have a structure including two or more multi-core processors.

The processor 10a may be any one of integrated circuits (ICs) such as CPUs (Central Processing Units), MPUs (Micro Processing Units), APUs (Accelerated Processing Units), DSPs (Digital Signal Processors), ASICs (Application Specific Integrated Circuits), and FPGAs (Field Programmable Gate Arrays), or combinations of two or more of these ICs.

The graphic processing device 10b controls screen-displaying on an output device such as a monitor display among the IO device 10f. Further, the graphic processing device 10b may have a configuration serving as an accelerator that executes a machine learning process and an inference process using a machine learning model. Examples of the graphic processing device 10b are various ICs such as Graphic Processing Units (GPUs), APUs, DSPs, ASICs, and FPGAs.

The memory 10c is an example of a hardware device that stores various pieces of data and information such as a program. An example of the memory 10c is one of a volatile memory such as a Dynamic Random Access Memory (DRAM) and a non-volatile memory such as a persistent Memory (PM) or both.

The storing device 10d is an example of a hardware device that stores information such as various data, programs, and the likes. Examples of the storing device 10d may be various storing devices including a magnetic disk device such as a Hard Disk Drive (HDD), a semiconductor drive device such as a Solid State Drive (SSD), a nonvolatile memory, and the like. The non-volatile memory may be, for example, a flash memory, a Storage Class Memory (SCM), a Read Only Memory (ROM), and the like.

The storing device 10d may store a program 10h (drug efficacy predicting program) that implements all or a part of various functions of the computer 10.

For example, the processor 10a of the drug efficacy predicting device 1 may achieve a drug efficacy predicting function to be described below by expanding the program 10h stored in the storing device 10d on the memory 10c and executing the expanded program 10h.

The IF device 10e is an example of a communication IF that controls connections and communications between the computer 10 and other devices. For example, the IF device 10e may include an applying adapter conforming to Local Area Network (LAN) such as Ethernet® or optical communication such as Fibre Channel (FC). The applying adapter may be compatible with either or both of wireless and wired communication schemes.

For example, the drug efficacy predicting device 1 may be communicably connected to another non-illustrated information processing device via the IF device 10e and a network. The program 10h may be downloaded from the network to the computer 10 via the communication IF and stored in the storing device 10d.

The IO device 10f may include one or both of an input device and an output device. Examples of the input device include a keyboard, a mouse, and a touch panel. Examples of the output device include a monitor, a projector, and a printer. The IO device 10f may include, for example, a touch panel that integrates an input device and an output device with each other.

The reader 10g is an example of a reader that reads information of data and programs recorded on a recording medium 10i. The reader 10g may include a connecting terminal or device to which the recording medium 10i can be connected or inserted. Examples of the reader 10g include an applying adapter conforming to, for example, Universal Serial Bus (USB), a drive apparatus that accesses a recording disk, and a card reader that accesses a flash memory such as an SD card. The program 10h may be stored in the recording medium 10i. The reader 10g may read the program 10h from the recording medium 10i and store the read program 10h into the storing device 10d.

Examples of the recording medium 10i illustratively include a non-transitory computer-readable recording medium such as a magnetic/optical disk, and a flash memory. Examples of the magnetic/optical disk include a flexible disk, a Compact Disc (CD), a Digital Versatile Disc (DVD), a Blu-ray disk, and a Holographic Versatile Disc (HVD). Examples of the flash memory include a semiconductor memory such as a USB memory and an SD card.

The HW configuration of the computer 10 described above is exemplary. Accordingly, the computer 10 may appropriately undergo increase or decrease of HW devices (e.g., addition or deletion of arbitrary blocks), division, integration in an arbitrary combination, or addition or deletion of the bus.

Example of Functional Configuration:

As illustrated in FIG. 1, the drug efficacy predicting device 1 may illustratively include the functions as a first data generating unit 2, a first predicting model training unit 3, a first predicting model 4, a second data generating unit 5, an inferring unit 6, and a second predicting model 7. These functions may be accomplished by hardware of the computer 10 (see FIG. 2).

The first data generating unit 2 generates data to be used for training for the first predicting model 4. Hereinafter, a plurality of such data are referred to as a "first data set". From the first data set, the first data generating unit 2 derives another group of data items, hereinafter referred to as a "second data set," which is actually input into the first predicting model 4.

Here, the first predicting model 4 is a machine learning model that predicts a difference in IC50s. The first predicting model 4 is not specific to any particular experimental system $S_m$ and can be shared across different experimental systems. An example of the first predicting model 4 is a neural network. The neural network may be implemented in hardware circuitry, or may be a virtual network generated by means of software that connects layers virtually constructed on a computer program by the processor 10a. A neural network may be abbreviated to a "NN". In addition, hereinafter a difference **between** IC50 values is sometimes represented by a symbol $IC_{diff}$.

An item set including an experimental system $S_m$, a drug $D_i$, and a cell line $C_j$ may be expressed by the symbol V. An experimental system $S_m$, a drug $D_i$ and a cell line $C_j$ correspond to data related to experimental conditions. Here, the experimental system refers to the overall combination of conditions, such as a culture media for cell culture and a method for measuring cell viability excluding the main conditions, namely candidates for anticancer agents and cell lines. For example, the culture media may include "DMEM (Dulbecco's Modified Eagle Medium" and "Roswell Park Memorial Institute(RPMI) 1640", and examples of the method for measuring the viability of cells are "luminescence firefly luciferase method (Luciferase Assay)" and "trypan blue-exclusion test (TBET)". Hereinafter, each of the experimental system $S_m$, the drug $D_i$, and the cell line $C_j$ is sometimes referred to as an "item" and an "item condition". An item set V does not have to include all three items of the experimental system $S_m$, the drug $D_i$ and the cell line $C_j$, and may include at least one of the items.

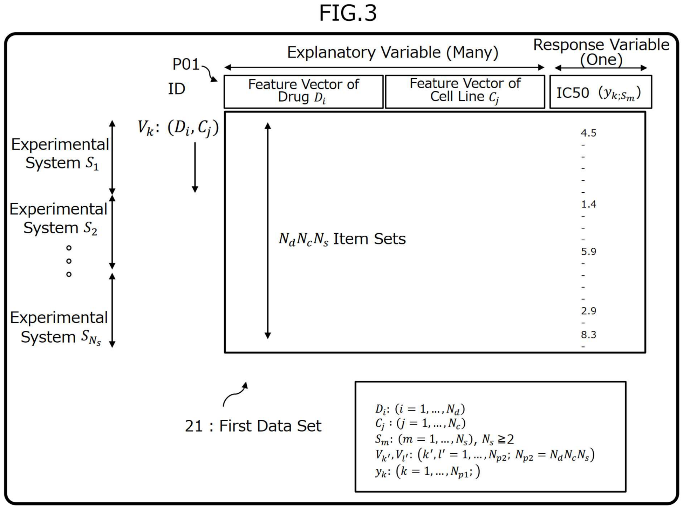

FIG. 3 is a diagram schematically illustrating a first data set of the drug efficacy predicting device 1 according to the embodiment.

In FIG. 3, the first data set is designated by the reference number 21, and is hereinafter simply referred to as "the first data set 21."

In FIG. 3, the first data set 21 is represented by a table format (see, the reference sign P01 in FIG. 3) in which the IC50 value is associated with the combination $V_k$ of the drug $D_i$ and the cell line $C_j$ for each experimental system $S_m$. Each value (element) in the table may be referred to as a "field".

The first data set 21 includes drugs $D_i$ (where, i = 1 , ... , $N_d$). The first data set 21 includes cell lines $C_j$ (where, j=1,..., $N_c$). The first data set 21 includes experimental systems $S_m$ (where, m=1,..., $N_s$, where Ns is an integer of two or more).

A combination $(D_i, C_j)$ of the drug $D_i$ and the cell line $C_j$ is represented by the reference symbol $V_k$. The symbols k' and l' each represent an item set associated with an experimental system $S_m$.

The value of k' is k'=1,..., $N_{p2}$, and the value of l' is 1'=1,..., $N_{p2}$. $N_{p2}$ is given by the equation $N_{p2}=N_d \times N_c \times N_s$ The IC50 value is represented by a reference symbol $y_k$ (where k=1,..., $N_{p1}$). $N_{p1}$ is given by the equation $N_{p1}=N_d \times N_c$.

The first data set 21 includes a measured IC50 value corresponding to a combination of a drug $D_i$, a cell line $C_j$, and an experimental system $S_m$.

In the first data set 21 illustrated in FIG. 3, some item sets have measured IC50 values and a field of an item set without a measured value is represented by the symbol "-" and may be referred to as an "empty field".

The first data generating unit 2 extracts a pair $(y_{k'}, y_{l'})$ of measured IC50 values from the first data set 21 and calculates a difference $IC_{diff}$. Then, the first data generating unit 2 generates the second data set 22 by associating the calculated difference $IC_{diff}$ with the corresponding cell lines $C_j$ and drugs $D_i$ corresponding to the respective extracted measured values $(y_{k'}, y_{l'})$. Here, $y_{k'}$, and $y_{l'}$ are measured values of IC50 for the item sets $V_{k'}$, and $V_{l'}$, respectively.

In extracting a pair $(y_{k'}, y_{l'})$ of the measured IC50 values, the first data generating unit 2 preferably selects the values such that they are both measured in the same experimental system $S_m$. However, selection of measured values of different experimental systems is also permitted.

In addition, the first data generating unit 2 generates a pair $(V_{k'}, V_{l'})$ of item sets, where each item set includes a drug D and a cell line C, and the two item sets share at least one of these items. An item set may further include the experiment system $S_m$.

A pair of item sets corresponds to two data combinations with the same experimental system, where the two data combinations share at least one common item, either the drug or the cell line.

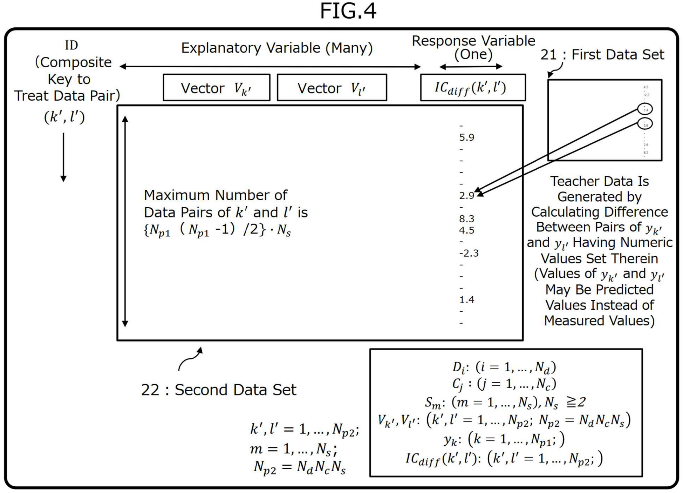

FIG. 4 is a diagram schematically illustrating a second data set of the drug efficacy predicting device 1 according to the embodiment.

In FIG. 4, the second data set is designated by the reference number 22. Hereinafter, the second data set is sometimes referred to as the second data set 22. In addition, FIG. 4 schematically illustrates the data set 22 for the experimental system $S_m$.

A pair (data pair) of an item set k' and an item set l' associated with the experimental system $S_m$ is represented by ID(k', l'). The ID serves as a composite key identifying the data pair.

The symbols k' and l' are indices that specify a combination of the drug $D_i$, the cell line $C_j$, and the experimental system $S_m$.

In FIG. 4, the second data set 22 is illustrated in the form of a table. Each row associates a feature vector indicating an item set $V_{k'}$, a feature vector indicating an item set $V_{l'}$, and the difference $IC_{diff}(k', l')$ between the measured IC50 values of the item sets k' and l', with the data pair (k', l') in the experimental system $S_m$. The ranges of k' and l' are both 1, ..., $N_{p2}$, and the relationship $Np2 = N_d \times N_c \times N_s$ is satisfied.

Each of the feature vectors for item sets $V_{k'}$ and $V_{l'}$ includes a feature vector representing the drug $D_i$ and a feature vector representing the cell line $C_j$. For the experimental system $S_m$, a feature vector - for example a one-hot vector - may be used.

In the second data set 22 illustrated in FIG. 4, each row represents a single difference value $IC_{diff}(k', l')$ calculated from the measured IC50 values of item sets k' and l'. A field marked with the symbol "-" indicates that no IC50 measured value is available for that combination. Such missing values are intended to be predicted and filled by the drug efficacy predicting device 1.

The second data set 22 illustrated in FIG. 4 generated as a single set including all experimental systems ($S_m$). Even when the underlying drug-cell-line pair corresponding to data pairs (k', l') is the same, the measured IC50 values- and thus $IC_{diff}(k', l')$-may differ across experimental systems $S_m$.

The maximum number of data pairs (k', l') that can be formed in the second data set 22 is given by $\{N_{p1}(N_{p1}-1)/2\}$ $\cdot N_s$, where $Np1 = Nd \times Nc$ and Ns is the number of experimental systems. This represents the theoretical maximum; in practice, the actual number of available data pairs may be smaller because some IC50 values are missing.

The first predicting model training unit 3 trains the first predicting model 4, using the second data set as training data. The training is performed on all available data pairs (k', l') contained in the second data set 22, with the difference values $IC_{diff}(k', l')$ serving as the response variable.

The trained first predicting model 4 is then applied to data pairs (k', l') for which no measured IC50 values are available in the second data set 22, so that predicted difference values $IC_{diff}(k', l')$ are obtained.

In this prediction process, the feature vectors of the drugs $D_i$ and the cell lines $C_j$ of the data pair (k', l') are input into the first predicting model 4, and the model outputs the difference value $IC_{diff}(k', l')$ between the IC50 value of the item set k' and that of the item set l'.

In this context, the feature vector V of an item set represents a composite vector including (i) a feature vector of the drug Di, (ii) a feature vector of the cell line Cj, and (iii) a feature vector indicating the experimental system $S_m$, for example in the form of a one-hot vector. Thus, each item set $v_{k'}$ or $v_{l'}$ corresponds to a unique combination of a drug, a cell line, and an experimental system, and is expressed as the feature vector V = (Di, Cj, $S_m$).

The first predicting model training unit 3 trains the first predicting model 4 by using the feature vectors of the item set k' and the feature vectors of the item set l' as explanatory variables and using the difference $IC_{diff}(k', l')$ between the IC50 values as a response variable.

In this training, the difference $IC_{diff}(k', l')$ is calculated from measured IC50 values of the corresponding item sets, as $IC_{diff}(k', l') = Y_i - Y_j$.

The first predicting model training unit 3 further provide training data obtained from different experimental systems $S_m$ for the same item sets k' and l', thereby enabling the first predicting model 4 to learn common properties of $IC_{diff}$ that are independent of the experimental system. The training may also be performed on difference values $IC_{diff}$ of various item sets within the same experimental system $S_m$.

The first predicting model 4 learns the commonality and the variation of drug efficacy across different experimental systems by using data from various experimental systems as training data. For data pairs within the same experimental system, the model learn dependencies on items such as drug and cell line. In addition, by using multiple data having the same item set but from different experimental systems, the model can learn similarities and the differences arising solely from changes in the experimental system.

The second data generating unit 5 completes the second data set 22 by predicting the difference $IC_{diff}(k', l')$ between IC50 values for row (data pair) lacking measured differences, , using the first predicting model 4.

The second data generating unit 5 generates a third data set 23 for the training the second predicting model 7, using the completed second data set 22 and the first data set 21 as described above.

An example of the second predicting model 7 may be implemented as a neural network. The neural network may be realized in hardware circuitry or in software by connecting layers implemented in a computer program executed by the processor 10a.

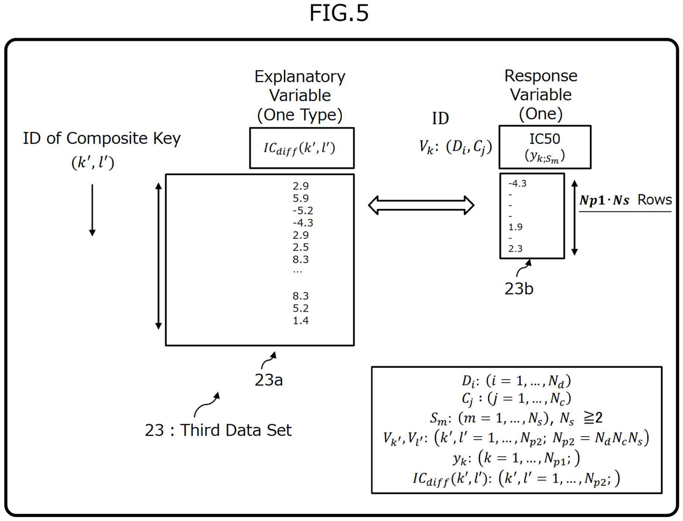

FIG. 5 is a diagram illustrating a third data set 23 in the drug efficacy predicting device 1 according to one embodiment.

The third data set 23 illustrated in FIG. 5 includes a paired-data domain 23a and a single-data domain 23b. The paired-data domain 23a is used as explanatory variables for training the second predicting model 7, while the single-data domain 23b is used as the response variable.

The pair data domain 23a is represented in a table format that associates a difference $IC_{diff}(k', l')$ between a measured value of an IC50 of the item set k' and a measured IC50 value of the item set l' with a data pair (k', l') of the item set k' and the item set l' in the experimental system $S_m$.

The paired-data domain 23a includes all $IC_{diff}(k', l')$ values contained in the second data set 22. For any data pair lacking a measured IC50 difference, a value predicted by the first predicting model 4 is substituted.

In the pair data domain 23a, the maximum number of data pairs (the number of rows) of k' and l' is $\{N_{p1}(N_{g1}-1)/2\}$ $\cdot N_s$. Numeric values are set for all the fields in the third data set 23, so that it contains no empty fields.

In addition, the single data domain 23b is represented in a table format that associates the IC50 value value $(y_{k;Sm})$ with the item set $V_k(D_i, C_j)$.

The number of rows in the single data domain 23b is $N_{p1} \cdot N_s$. In the third data set 23, the data of the pair data domain 23a and the data of the single data domain 23b are not in a one-to-one correspondence.

Some rows of the single data domain 23b have measured values. The second predicting model 7 infers values so as to satisfy a difference relationship between the data of the pair data domain 23a and the data of the single data domain 23b.

The second data generating unit 5 causes the second predicting model 7 to predict a value in rows ("-") lacking measured values, by reconciling the paired-data domain 23a and the single-data data domain 23b.

The inferring unit 6 trains the second predicting model 7 using the third data set 23, and outputs a response variable y (the inferred IC50 value).

The second predicting model 7 estimates an unknown $y_{k;Sm}$ based on many $IC_{diff}(k', l')$ and a subset of measured values of $y_{k'}$ and $y_{l'}$. After parameters are obtained through training, the drug efficacy predicting device 1 infers the response variables (overall drug efficacy) simultaneously for a set, rather than inference of each individual response variable.

The second predicting model 7 corresponds to the second machine learning model that predicts drug efficacy (y) of each of two or more data items from the predicted difference $IC_{diff}$.

The second predicting model 7 is trained using (i) the differences between measured IC50 values and (ii) feature vectors representing experimental conditions (at least one of an experimental system, a drug, and a cell line) as explanatory variables and using the measured IC50 values (drug efficacy) as a response variable.

All the pairs (k', l') the paired-data domain 23a of the third data set 23 are completed with differences based on measured or predicted values. It is expected that the overall pair-data domain 23a and all the actually measured values $y_{l'}$ of the single data domain 23b may deviate from each other in terms of differences. The second predicting model 7 functions as a machine learning model that absorbs this deviation. In other words, the second predicting model 7 is modeled by the following expression (1) in which a noise term is represented by $\varepsilon(k', l')$, and a scale and a bias peculiar to the experimental system $S_m$ are represented $a_m$, $b_m$, respectively.

$$IC_{diff}(k', l') = b_m + a_m(y_{k'} - y_{l'}) - \varepsilon(k', l') \qquad (1)$$

Accordingly, the cost function is defined as shown below, and the term F is minimized, for example, the steepest descent method, based on $IC_{diff}(k', l')$ and the measured value $y_{k'}$, $y_{l'}$.

[Expression 1]

$$F = \sum_{(k',l')} \epsilon(k', l')^2$$

$$= \sum_{(k',l')} \left( b_m + a_m(y_{k'} - y_{l'}) - IC_{diff}(k', l') \right)^2$$

If the drug efficacy y has an actual measured value, the term y is fixed at the measured value. However, if being not measured, the drug efficacy y is treated as a variable to be inferred. That is, the value y is obtained by iterative improvement starting with a random value. Model learning is carried out through minimizing F and the coefficients $b_m$, $a_m$ (where, $m=1,...,N_s$) are simultaneously inferred. This method obtains all the solutions at once.

In the drug efficacy predicting device 1, a value $y_{k'}$ is obtained for each experimental system $S_m$. The overall drug efficacy may then be determined based on the prediction from a particular experimental system, or by using the average across all experimental systems. This determination can be modified as appropriate, and the way drug efficacy is judged from these inferences is left to the discretion of the user.

As described above, the second data generating unit 5 generates the third data set 23 based on the item sets of the same experimental system S. The generation of the third data set 23 is carried out for each of the multiple types of experimental systems S. The $IC_{diff}$ obtained for the multiple experimental systems $S_m$ may be generalized and expressed as $IC_{diff}(k', l'; S_m)$.

Operation:

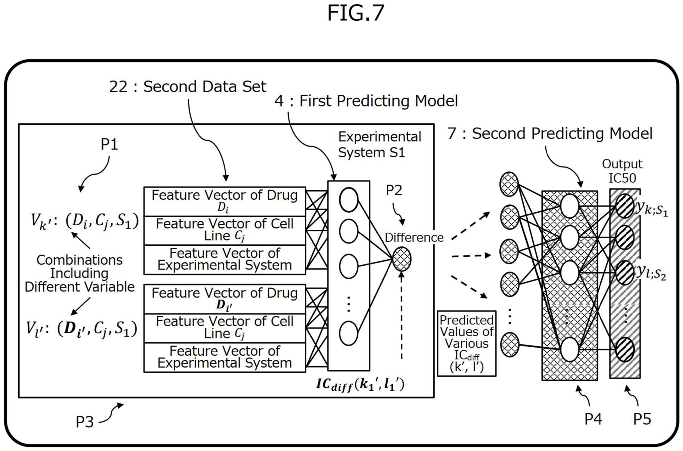

Description will now be given of a process performed in the drug efficacy predicting device 1 of the one embodiment having the above configuration along the flow chart (Steps A1-A6) with reference to FIG. 7, which illustrates an overview of the process performed in the drug efficacy predicting device 1.

In Step A1, the first data generating unit 2 generates the second data set 22 by extracting a pair of item sets (conditions) from among multiple item sets of the same experimental system included in the first data set 21 and calculating a difference $IC_{diff}$ between the measured values of the IC50s corresponding to the item sets. The second data set 22 is used as training data for training the first predicting model 4.

In Step A2, the first predicting model training unit 3 trains the first predicting model 4, using the second data set 22 generated in Step A1, namely, all the pairs of item sets and the differences $IC_{diff}$ of the IC50s prepared in Step A1.

The second data set 22 is input into the first predicting model 4, which is a machine learning model that predicts the difference in IC50s. The second data set 22 includes a pair of the item set ($V_{k'}$ and $V_{l'}$) consisting of a drug and a cell line for each experimental system (see the reference sign P1 in FIG. 7). These items $V_{k'}$ and $V_{l'}$ are different combinations of variables.

For example, $V_{k'}$ is assumed to have the measured value $y_{k';s1}$ of the IC50 under the condition ($D_i$, $C_j$, $S_1$). In addition, $V_1$, is assumed to have the measured value $y_{l';s1}$ of the IC50 of under the condition ($D_{i'}$, $C_j$, $S_1$), for example. In this case, the difference in the IC50s can be expressed as $IC_{diff}$ ( k', l' ; $S_1$) $=y_{k';S1}-y_{l';S1}$.

In Step A3, the second data generating unit 5 predicts the difference $IC_{diff}(k',l';S_m)$ of the IC50 values of a row (data set, input) lacking measured IC50 values, using the first predicting model 4.

The first predicting model 4 outputs a single difference value, $\{IC_{diff} (k', l')\}$ for the input pair of $V_{k'}$ and $V_{l'}$ (see the reference sign P2 in FIG. 7). That is, the first predicting model 4 performs regression prediction of the value of the difference in IC50s. The same process is performed on each of the multiple experimental systems (see the reference sign P3 in FIG. 7).

In this way, the second data generating unit 5 supplements the second data set 22 by predicting differences between IC50 values, using the first predicting model 4. This completes the second data set 22.

In Step A4, the second data generating unit 5 generates the third data set 23, using the completed second data set 22 and the first data set 21 (first data group) as described above.

In Step A5, the inferring unit 6 trains the second predicting model 7, using the third data set 23 (see the reference sign P4 in FIG. 7).

In Step A6, the inferring unit 6 outputs the response variable (inferred value of the IC50) y, using the second predicting model 7 (see the reference sign P5 in FIG. 7).

Effect:

As described above according to a drug efficacy predicting device 1 serving as an example of the embodiment, the second data generating unit 5 generates the third data set 23 that includes a single data domain 23b having the feature vector of the experimental system $S_m$ and the difference $IC_{diff}(k', l';S_m)$ and the single data domain 23b that associates the value ($y_k;S_m$) of the IC50 with the data set $V_k$ ($D_i$, $C_j$).

Then, the inferring unit 6 generates the second predicting model 7, using the third data set 23, and outputs the inferred value of the IC50 (y), using the second predicting model 7.

By using item sets of the same experimental system in training the second predicting model 7 as described above, the item set of the same experimental system can be reflected in inferring the IC50 value. Furthermore, in inferring the value of the IC50 serving as an index representing drug efficacy, learning the difference between a pair of response variables makes it possible to carry out inference using data obtained by integrating data of the same experimental system.

Furthermore, the first data generating unit 2 generates the second data set 22 that registers therein the difference of the IC50s between the data pairs of the two data sets of the same experimental system. The first predicting model training unit 3 trains the first predicting model 4, using the second data set 22. Then, the second data generating unit 5 completes the second data set 22 by predicting the difference $IC_{diff}(k',l')$ between IC50 values of a row (item set) lacking the difference between the measured IC50 values, using the first predicting model 4.

Here, the first predicting model 4 (node) that learns and predicts the difference of the measured IC50 values functions as a kind of auxiliary line for the second predicting model 7 to grasp and learn the relationship (structure) among data.

In the present drug efficacy predicting device 1, as preprocessing before the prediction of the objective value $y_i$ (IC50), the first predicting model training unit 3 first causes the first predicting model 4 to learn the difference $IC_{diff}$ (k, l)$=y_i-y_j$ from the pair of $y_i$ and $y_j$ (both of which are measured values). The second data generating unit 5 completes the second data set 22 by predicting a predicted value of the difference $IC_{diff}$ (k,l) of an unknown condition, using the first predicting model 4 learned as the above. Then, the inferring unit 6 predicts the individual values $y_i$ and $y_j$ of IC50s from the multiple prediction results $IC_{diff}$, using the second predicting model 7.

A difference in IC50s is considered to be an essential variable with less fluctuation, so that the first predicting model 4 can stably infer the difference in IC50. Therefore, the first predicting model 4 directly learns the interrelationship between items (factors, such as a drug and a cell line, that directly determine IC50) and an experimental system and also an effect of the interrelationship on the IC50 value. Consequently, the IC50 value can be inferred more accurately.

In convolution in deep learning, the feature of an image can be easily grasped by assigning the total sum of correlated pixels of an image data to one node and replacing the presence of a certain data structure with a numeric value. It can be considered that the parameters in the second predicting model 7 act similarly to such a convolutional node.

The present drug efficacy predicting device 1 models the specificity of an experimental system according to the actual conditions. For example, in general, about 500 drugs can be acceptable at the maximum and the dispersion of the similarly enables hierarchical clustering.

Furthermore, in general, about 1,000 cell lines can be acceptable at the maximum and the dispersion of the similarly enables hierarchical clustering also for the cell lines.

In contrast, as few as two to ten experimental systems and large variation of solvents make it difficult to define the similarity.

As a method for interpreting the IC50 value, the IC50 values can be comparable as far as the experimental system is the same. The values of the IC50s of different experimental systems are measured for evaluation of the drug efficacy. For the above, although varying the experimental systems does not guarantee that the values of the IC50s coincide with each other, an assumption seems to be accepted which in comparing different drugs and different cell lines, the result of evaluating the magnitude relationship of the IC50 values would be stable.

The present drug efficacy predicting device 1 adopts such domain knowledge by giving a specific expression (see the above Expression (1)) of the $IC_{diff}$ to a model. This means that, despite modeling of the specificity of an experimental system according to the actual conditions, the evaluation of the present drug efficacy predicting device 1 is different from simply adopting, as an explanatory variable, an experimental system to a machine learning model.

In the same experimental system, the IC50 values of different item conditions (drugs, cell lines) can be compared, and drug efficacy is evaluated by comparing multiple conditions. When the experimental system is changed, the measured IC50 values are compared and evaluated using the new experimental system as a common system. Accordingly, the magnitude relationship of the measured IC50 values is regarded as a numeric value representing the essential drug efficacy, which does not largely change if the experimental system is varied.

When the difference is introduced, as a variable, into a machine learning model (second predicting model 7), the essential information can be learned and an accurate IC50 value can be predicted without being affected by noise.

By training the difference as a quantity that is not affected very much by experimental systems, training data of different experimental systems with the same model makes it easier to grasp the correlation between variables than learning the IC50s themselves, which enables stable prediction.

This difference is not expected to largely change if the system (experimental system) is changed, but the scale of the difference may vary. Since the contents of a system are learned by including them as variables in the input, system-related differences, such as a difference in scale, can also be learned.

Miscellaneous:

Each configuration and processes of the present embodiment may be selected and omitted according to the requirement and may be appropriately combined.

The disclosed technique is not limited to the above-described embodiment and can be variously modified without departing from the scope of the present embodiment.

For example, as a method of using data from multiple experimental systems, the first data set 21 may be divided into multiple regions, and each divided region may be sequentially used (processed).

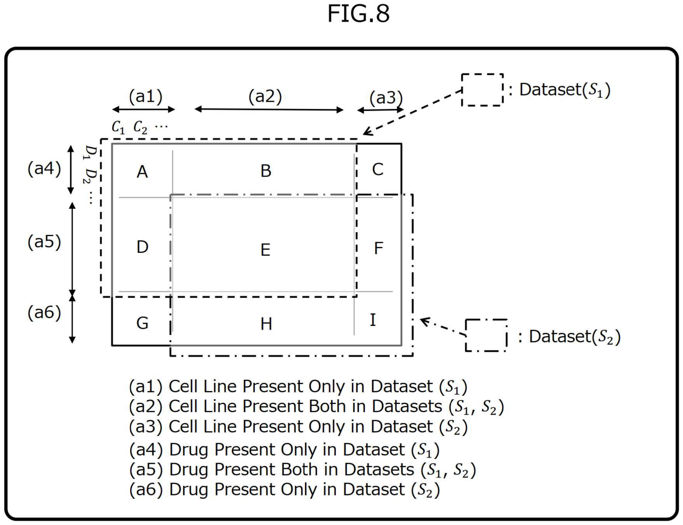

FIG. 8 is a diagram illustrating an example of a state where the first data set 21 is divided into two regions and two experimental systems $S_1$ and $S_2$ exist.

In FIG. 8, the first data set 21 is divided into three regions (a1) to (a3) in the row direction, and is also divided into three regions (a4) to (a6) in the column direction.

The region (a1) indicates a cell line present only in Dataset ($S_1$). The region (a2) indicates a cell line present in both Datasets ($S_1$, $S_2$). The region (a3) indicates a cell line present only in Dataset ($S_2$).

The region (a4) indicates the drug present only in Dataset ($S_1$). The region (a5) indicates the drug present in both Datasets ($S_1$, $S_2$). The region (a6) indicates the drug present only in Dataset ($S_2$).

The region where the region (a1) and the region (a4) overlap is represented by a region A, the region where the region (a1) and a region (a5) overlap is represented by a region D, and the region where the region (a1) and the region (a6) overlap is represented by a region G. The region where the region (a2) and the region (a4) overlap is represented by a region B, the region where the region (a2) and the region (a5) overlap is represented by a region E, and the region where the region (a2) and the region (a6) overlap is represented by a region H. The region where the region (a3) and the region (a4) overlap is represented by a region C, the region where the region (a3) and the region (a5) overlap is represented by a region F, and the region where the region (a3) and the region (a6) overlap is represented by a region I.

In the above-described embodiment, the first data set 21 is regarded as a single region, not distinguishing the respective regions of the first data set 21. Specifying two fields each having a value of the IC50 from among the fields of all the regions, a pair difference of which can be calculated can be obtained (however, the systems of the two fields are assumed to be the same).

The first data generating unit 2 generates data sets of the second data set 22 by capturing many possible pairs in the first data set 21 (the second data set 22 may have multiple systems). The first predicting model training unit 3 learns the second data set 22 to generate the first predicting model 4, and the second data generating unit 5 predicts an unknown difference $IC_{diff}$, using the first predicting model 4. The second data generating unit 5 generates the third data set 23 by adding predicted values of the differences $IC_{diff}$ and the inferring unit 6 infers an unknown IC50 value, using the second predicting model 7. The method described in the above embodiment may be referred to as a scheme I.

On the other hand, the present modification divides the entire region of the first data set 21 into multiple (T) groups in terms of an experimental condition and the similarity of the systems. In the example illustrated in FIG. 8, the first data set 21 is divided into three (T=3) groups of: {E}, {B,D,F,H}, and {C,G}.

First, the region E of the group {E}, which includes measured values of the most systems, is selected and the values of the IC50s of all the empty fields are inferred in the same manner as the above scheme I.

After that, the respective empty fields of the regions B,D,F,H adjoining the region E are filled. The inferred IC50 values of the region E as the above result are also regarded as experimental values, and a set in which all the data of IC50s in the region E and the measured values of the IC50s in the regions B, D, F, and H are merged considered. After that, like the scheme I, supervised learning is performed on pairs in the merged set using the traning data ($IC_{diff}$), and the values of the IC50s of all the empty fields are predicted.

Next, the empty fields of the regions C and G not directly adjoining the region E are filled. Then, a set in which all the data of the above regions E,B,D,F, and H and the traning data of the regions C and G are merged is considered. After that, the values of the IC50s of all the empty fields are predicted in the same manner.

As a result, the values of the IC50s of all the T regions can be obtained by the inference. Such a method of combining the region-division and the sequential use of the first data set 21 may be referred to as a scheme II.

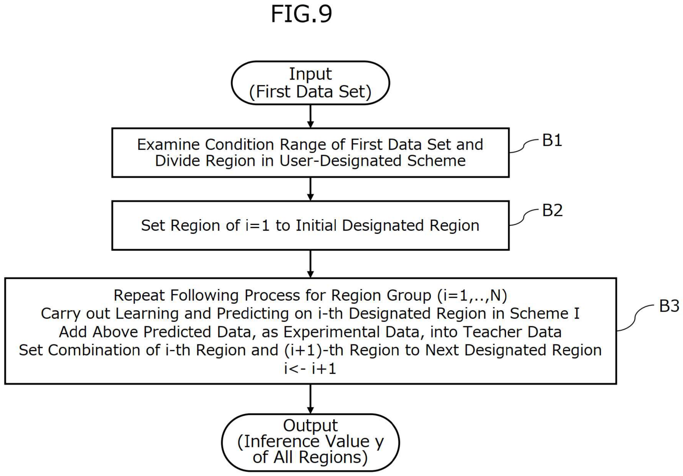

FIG. 9 is a flow chart illustrating a process performed in the drug efficacy predicting device 1 according to a modification to the one embodiment (Steps B1-B3).

The first data set 21 is input. In Step B1, the condition range of the first data set is examined, and the first data set 21 is divided into multiple regions according to a user-designated scheme (i=1, ..., N).

In Step B2, the region with i=1 is set to the initial designated region.

In Step B3, the following process is repeatedly executed until the region group (i=1,..., N) undergo the process. Specifically, learning and predicting in the scheme I are carried out on the i-th designated region and the predicted value obtained by the above prediction is regarded as an experimental value and added to the training data. Then, the i-th region and the (i+1)-th region are combined into the next designated region (i=i+1). The inferred values y of all the regions are output, and the process ends.

The above-described embodiment and modification describe examples that use an IC50 as a value representing drug efficacy, but the value is not limited to the IC50 and may alternatively be a value except for an IC50.

In addition, the above-described embodiment and modification obtain the difference ($IC_{diff}$) of drug efficacy (IC50s) by calculating differences of two item sets ($V_k$, $V_l$) each serving as a data pair. However, the manner of obtaining of the drug efficacy is not limited to this. Alternatively, the difference ($IC_{diff}$) of drug efficacy (IC50s) may be calculated on the basis of the values of the respective IC50 of three or more item sets.

The present embodiment can be implemented and carried out by those ordinary skilled in the art referring to the above disclosure.

In any of the above aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein. A computer program embodying the invention may be stored on a non-transitory computer-readable medium, or it could, for example, be in the form of a signal such as a downloadable data signal provided from an Internet website, or it could be in any other form.

[Reference Sign List]

1    drug efficacy predicting device
2    first data generating unit
3    first predicting model training unit
4    first predicting model
5    second data generating unit
6    inferring unit
7    second predicting model
10   computer
10a  processor
10b  graphic processing device
10c  memory
10d  storing device
10e  IF device
10f  IO device
10g  reader
10h  program
10i  recording medium
10j  bus
21   first data set
22   second data set
23   third data set
23a  pair data domain
23b  single data domain

**Claims**

1.  A drug efficacy predicting program for causing a computer (10) to execute a process comprising:

    predicting, by using a first machine learning model (4), a difference in drug efficacy between one or more of two data combinations under a same experimental system, each of the two data combinations including at least one common item among items of a drug and a cell line; and
    predicting, by using a second machine learning model (7), one or more drug efficacies of two or more data using the predicted difference.

2.  The drug efficacy predicting program according to claim 1, wherein
    the first machine learning model (4) is trained, using feature vectors of each of the two data combinations as an explanatory variable and using a difference in measured value of the drug efficacy between the two data combinations as a response variable.

3.  The drug efficacy predicting program according to claim 2, wherein
    the second machine learning model (7) is trained, using the difference in the measured value of the drug efficacy and the feature vectors of each of the two or more data combinations as explanatory variables and using the measured value of the drug efficacy as a response variable.

4.  A computer-implemented method for predicting drug efficacy comprising:

    predicting, by using a first machine learning model (4), a difference in drug efficacy between one or more of two data combinations under a same experimental system, each of the two data combinations including at least one common item among items of a drug and a cell line; and
    predicting, by using a second machine learning model (7), one or more drug efficacies of two or more data using the predicted difference.

5.  The computer-implemented method according to claim 4, wherein
    the first machine learning model (4) is trained, using feature vectors of each of the two data combinations as an explanatory variable, and using a difference in measured value of drug efficacy between the two data combinations as a response variable.

6.  The computer-implemented method according to claim 5, wherein

the second machine learning model (7) is trained, using the difference in the measured value of the drug efficacy and the feature vectors of each of the two or more data combinations as explanatory variables and using the measured value of the drug efficacy as a response variable.

7. A drug efficacy predicting device comprising a controller configured to:

predict, by using a first machine learning model (4), a difference in drug efficacy between one or more of two data combinations under a same experimental system, each of the two data combinations including at least one common item among items of a drug and a cell line; and

predict, by using a second machine learning model (7), one or more drug efficacies of two or more data using the predicted difference.

8. The drug efficacy predicting device according to claim 7, wherein
the first machine learning model (4) is trained, using feature vectors of each of the two data combinations as an explanatory variable, and using a difference in measured value of drug efficacy between the two data combinations as a response variable.

9. The drug efficacy predicting device according to claim 8 wherein
the second machine learning model (7) is trained, using the difference in the measured value of the drug efficacy and the feature vectors of each of the two or more data combinations as explanatory variables and using the measured value of the drug efficacy as a response variable.

# FIG.1

FIG.2

# FIG.3

## FIG.4

ID
(Composite Key to Treat Data Pair)

$(k', l')$

Explanatory Variable (Many)

Response Variable (One)

21 : First Data Set

Vector $V_{k'}$    Vector $V_{l'}$    $IC_{diff}(k', l')$

- 5.9 - - - 2.9 - 8.3 4.5 - -2.3 - - - 1.4 - -

Maximum Number of Data Pairs of $k'$ and $l'$ is $\{N_{p1} \ (N_{p1} - 1) \ /2\} \cdot N_s$

Teacher Data Is Generated by Calculating Difference Between Pairs of $y_{k'}$ and $y_{l'}$ Having Numeric Values Set Therein (Values of $y_{k'}$ and $y_{l'}$ May Be Predicted Values Instead of Measured Values)

22 : Second Data Set

$k', l' = 1, \ldots, N_{p2};$
$m = 1, \ldots, N_s;$
$N_{p2} = N_d N_c N_s$

$D_i: (i = 1, \ldots, N_d)$
$C_j : (j = 1, \ldots, N_c)$
$S_m: (m = 1, \ldots, N_s), N_s \geqq 2$
$V_{k'}, V_{l'}: (k', l' = 1, \ldots, N_{p2}; N_{p2} = N_d N_c N_s)$
$y_k: (k = 1, \ldots, N_{p1}; )$
$IC_{diff}(k', l'): (k', l' = 1, \ldots, N_{p2}; )$

# FIG.5

Explanatory Variable (One Type)

$IC_{diff}(k', l')$

ID of Composite Key $(k', l')$

ID

$V_k: (D_i, C_j)$

Response Variable (One)

IC50 $(y_{k;s_m})$

2.9
5.9
-5.2
-4.3
2.9
2.5
8.3
...

8.3
5.2
1.4

-4.3
-
-
-
1.9
-
2.3

$Np1 \cdot Ns$ Rows

23b

23a

23 : Third Data Set

$$D_i: (i = 1, \dots, N_d)$$
$$C_j : (j = 1, \dots, N_c)$$
$$S_m: (m = 1, \dots, N_s), \ N_s \geqq 2$$
$$V_{k'}, V_{l'}: (k', l' = 1, \dots, N_{p2}; \ N_{p2} = N_d N_c N_s)$$
$$y_k: (k = 1, \dots, N_{p1};)$$
$$IC_{diff}(k', l'): (k', l' = 1, \dots, N_{p2};)$$

# FIG.6

Input
(Data-1)

↓

Generate Learning Data
(Second Data Set) Including Pair Serving
as Condition and Difference $IC_{diff}$ from
Pair from among Multiple Data Sets of
Same Experimental System ⟩—A1

↓

Generate First Prediction Model by
Learning All Prepared Pairs,
Using Second Data Set ⟩—A2

↓

Predict $IC_{diff}$ of Input Not Having
Measured Value
(i.e., Not Having Value of Response
Variable in Second Data Set)
Using Generated First Prediction Model ⟩—A3

↓

Generate Third Data Set Using Result of
Prediction and First Data Set ⟩—A4

↓

Generate Second Prediction Model
Using Third Data Set ⟩—A5

↓

Infer Response Variable y
With Second Prediction Model ⟩—A6

↓

Output
Inference Value y

## FIG.7

# FIG.8

(a1) Cell Line Present Only in Dataset ($S_1$)
(a2) Cell Line Present Both in Datasets ($S_1$, $S_2$)
(a3) Cell Line Present Only in Dataset ($S_2$)
(a4) Drug Present Only in Dataset ($S_1$)
(a5) Drug Present Both in Datasets ($S_1$, $S_2$)
(a6) Drug Present Only in Dataset ($S_2$)

# FIG.9

Input
(First Data Set)

Examine Condition Range of First Data Set and
Divide Region in User-Designated Scheme — B1

Set Region of i=1 to Initial Designated Region — B2

Repeat Following Process for Region Group (i=1,..,N)
Carry out Learning and Predicting on i-th Designated Region in Scheme I
Add Above Predicted Data, as Experimental Data, into Teacher Data
Set Combination of i-th Region and (i+1)-th Region to Next Designated Region
i<- i+1 — B3

Output
(Inference Value y
of All Regions)

Europäisches
Patentamt

European
Patent Office

Office européen
des brevets

# EUROPEAN SEARCH REPORT

**Application Number**

EP 25 21 2595

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SOTUDIAN SHAHABEDDIN ET AL: "Machine Learning for Pharmacogenomics and Personalized Medicine: A Ranking Model for Drug Sensitivity Prediction", IEEE/ACM TRANSACTIONS ON COMPUTATIONAL BIOLOGY AND BIOINFORMATICS, IEEE SERVICE CENTER, NEW YORK, NY, US, vol. 19, no. 4, 26 May 2021 (2021-05-26), pages 2324-2333, XP011916584, ISSN: 1545-5963, DOI: 10.1109/TCBB.2021.3084562 [retrieved on 2021-05-27] * Section 2.1, Equation (2), Equation (3), page 2 equation (1), page 2 equation (2), page 3 lemma 2.1 * | 1-9 | INV. G16H10/40 G16H50/20 G16H50/70 |
| Y | US 9 536 194 B2 (IBM [US]) 3 January 2017 (2017-01-03) * Abstract, col 2 lines 1-20, col 2 lines 5-8, col 2 lines 12-15, col 5 lines 1-30, col 6 lines 1-15, claims 1, 8 * | 1-9 | |
| Y | TYNES MICHAEL ET AL: "Pairwise Difference Regression: A Machine Learning Meta-algorithm for Improved Prediction and Uncertainty Quantification in Chemical Search", JOURNAL OF CHEMICAL INFORMATION AND MODELING, vol. 61, no. 8, 4 August 2021 (2021-08-04), pages 3846-3857, XP093341877, US ISSN: 1549-9596, DOI: 10.1021/acs.jcim.1c00670 Retrieved from the Internet: URL:https://pubs.acs.org/doi/pdf/10.1021/acs.jcim.1c00670> * Abstract, Introduction, Methods (Mathematical Formulation) * | 3,6,9 | TECHNICAL FIELDS SEARCHED (IPC) G16H |

The present search report has been drawn up for all claims

| Place of search | Date of completion of the search | Examiner |
|---|---|---|
| Munich | 20 February 2026 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 25 21 2595

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2026

| Patent document cited in search report | | Publication date | Patent family member(s) | | Publication date |
|---|---|---|---|---|---|
| US 9536194 | B2 | 03-01-2017 | US | 2015006438 A1 | 01-01-2015 |
| | | | US | 2015006439 A1 | 01-01-2015 |
| | | | US | 2017098062 A1 | 06-04-2017 |
| | | | US | 2017098063 A1 | 06-04-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021144619 A
- JP 2021039565 A
- US 20110173144
- JP 2019125045 A
- US 20200175380